(19)

Europäisches Patentamt
European Patent Office
Office européen des brevets

(11) **EP 2 090 660 A1**

(12) **EUROPÄISCHE PATENTANMELDUNG**

(43) Veröffentlichungstag:
 **19.08.2009 Patentblatt 2009/34**

(51) Int Cl.:
 ***C12P 5/02*** *(2006.01)*

(21) Anmeldenummer: 08101684.2

(22) Anmeldetag: **15.02.2008**

(84) Benannte Vertragsstaaten:
 **AT BE BG CH CY CZ DE DK EE ES FI FR GB GR HR HU IE IS IT LI LT LU LV MC MT NL NO PL PT RO SE SI SK TR**
 Benannte Erstreckungsstaaten:
 **AL BA MK RS**

(71) Anmelder: **Pfeifer & Langen Kommanditgesellschaft 50933 Köln (DE)**

(72) Erfinder:
 • **Lorenz, Markus Dr.**
  **52428 Jülich (DE)**
 • **Sievers, Michael Prof.**
  **38678 Clausthal-Zellerfeld (DE)**

(74) Vertreter: **Meyers, Hans-Wilhelm Patentanwälte von Kreisler-Selting-Werner Postfach 10 22 41 50462 Köln (DE)**

(54) **Verfahren zur Herstellung von Biogas unter Zugabe von Carbokalk**

(57) Verfahren zur Herstellung von Gas aus kohlenstoffhaltigen Materialien in einer Fermentationsanlage, **dadurch gekennzeichnet, dass** ein Hilfsstoff, welcher eine Calcium- und/oder Magnesiumverbindung umfasst, zugegeben wird.

Figur:

EP 2 090 660 A1

**Beschreibung**

Die vorliegende Erfindung betrifft ein Verfahren zur Herstellung von Gas aus kohlenstoffhaltigen Materialien in einer Fermentationsanlage und dessen Einsatz.

Biogas zählt zu den erneuerbaren Energieträgern. Es entsteht dort, wo kohlenstoffhaltiges, insbesondere organisches Material anaerob mikrobiell abgebaut wird. Biogas ist ein mit Wasserdampf und $CO_2$ gesättigtes Gasgemisch. Energetisch nutzbar ist sein Hauptbestandteil, das Methan, das typischerweise in Konzentrationen von 50 bis 70 Vol.-% vorkommt. Daneben kann es, je nach Ausgangsbedingungen, geringe Mengen an Schwefelwasserstoff, Ammoniak, Wasserstoff, Stickstoff und Spuren von niederen Fettsäuren und Alkoholen enthalten.

In einer landwirtschaftlichen Biogasanlage werden z. B. die Fäkalien der Nutztiere, aber auch Feldfrüchte wie Mais etc. eingesetzt. Für diese aber auch andere Biogasanlagen ist organisches Material mit hohem Wassergehalt ebenfalls gut geeignet. Hierzu zählen auch Zuckerrübenpflanzen mit und ohne Blätter, sowie Biomasse aus der Zuckerrübenverarbeitung und -aufarbeitung. Diese Biomasse kann dabei vor der Zugabe in eine Fermentationsanlage oder einen Fermenter hydrolysiert, vorversäuert oder siliert sein. In der Fermentationsanlage werden Mikroorganismen in einem Nährmedium (Fütterung) kultiviert. Der Fermenter kann dabei mesophil (bei ca. 39 °C) oder thermophil (bei ca. 55 °C) geführt werden.

Bei der Fermentation (Vergärung) organischer Stoffe unter anaeroben Bedingungen werden energiereiche, organische Substanzen als Substrat von Mikroorganismen umgesetzt. Nach dem heutigen Kenntnisstand erfolgt ein vierstufiger Abbau von organischen Stoffen bis zum Methan (siehe Mudrack K., Kunst, S., Biologie der Abwasserreinigung, Spektrum-Akademischer Verlag, 2003):

1. In einer Hydrolyse-Phase werden hochmolekulare, organische Stoffe durch Enzyme in lösliche Stoffe überführt.
2. In einer Versäuerungsphase werden von verschiedenen fakultativen und obligat anaeroben Bakterienarten kurzkettige organische Säuren, Alkohole, Wasserstoff und Kohlendioxid gebildet.
3. In der sogenannten Acetogenen Phase werden die in der Versäuerungsphase gebildeten organischen Säuren und Alkohole zu Essigsäure umgewandelt.
4. In der sogenannten Methanogenen Phase wird vornehmlich aus Essigsäure, Kohlendioxid und Wasserstoff Methan gebildet.

Als nicht gasförmiger Rückstand dieser Gärungsprozesse verbleibt ein sogenannter Ablauf, welcher einen Schlamm, welcher auch als Faulschlamm oder Gärrest bezeichnet wird, und eine flüssige Phase umfasst.

Das in einer Fermentationsanlage mikrobiell erzeugte Biogas lässt sich nach einer eventuellen Zwischenspeicherung energetisch nutzen. Besonders wirksam ist die Gasverwendung in dezentralen Blockheizkraftwerken.
Biogas kann auch in Agroindustrie und kommunaler Entsorgungswirtschaft erzeugt werden. Hier steht die Entsorgung neben der Energiegewinnung im Vordergrund.

Eine Fermentationsanlage, die zur Herstellung von Biogas dient, wird im Allgemeinen auch als Biogasanlage bezeichnet.
Zur Erzeugung von Biogas aus Biomasse aus der Landwirtschaft oder aus Bioabfällen ist aus der EP-B-0 934 998 ein sogenanntes Trockenfermentationsverfahren zur Methanisierung von halbfeuchter, schüttfähiger, stapelbarer oder stückigmachbarer, inokulierter Biomasse bekannt. Der in einem gasdichten Container eingelagerten Biomasse wird ein Impfmaterial zugesetzt und die so gebildete Reaktionsmasse wird unter Luftabschluss vergoren.
Ein Maß für die Kapazität einer Biogasanlage ist die sogenannte Raumzeitausbeute (RZA). Sie entspricht dem Massenstrom an Schlüsselkomponente dividiert durch das Reaktorvolumen und die Fermentationszeit.

Es gilt folgende Formel:

$$RZA = \frac{kg(OTS)}{m^3 * d}$$

OTS = organische Trockensubstanz
d = Tag (entspricht 24 Stunden)

Die durchschnittliche Raumzeitausbeute von Biogasanlagen beträgt etwa 4 kg/m³*d.

Überraschenderweise hat sich herausgestellt, dass ein Überschuss einer Calcium- und/oder Magnesiumverbindung als Hilfsstoff zu einer Fermentationsanlage, insbesondere einer Biogasanlage, zu einer höheren Raumzeitausbeute gegenüber konventionellen Fermentationsverfahren und einer besseren Abtrennung des Gärrestes führt.

Folglich liegt der Erfindung ein Verfahren zur Herstellung von Gas aus kohlenstoffhaltigen Materialien in einer Fermentationsanlage zugrunde, wobei ein Hilfsstoff, welcher eine Calcium- und/oder Magnesiumverbindung umfasst, in die Fermentationsanlage gegeben wird.
Dabei kann das Gas aus kohlenstoffhaltigen Materialien Biogas sein.

In einer Ausführungsform des erfindungsgemäßen Verfahrens wird der Zusatzstoff im Überschuss zugegeben. Ein Zusatz im Überschuss wird dabei durch Zugabe einer Menge erreicht, welche das Löslichkeitsprodukt der jeweiligen Verbindung deutlich überschreitet.

Ein Hilfsstoff im Sinne der Erfindung ist ein fester oder flüssiger Stoff oder ein Stoffgemisch aus festen und/oder flüssigen Stoffen.

In einer Ausführungsform des erfindungsgemäßen Verfahrens kann eine nicht leichtlösliche Calcium- und/oder Magnesiumverbindung als Hilfsstoff verwendet werden. Weiterhin können nicht leichtlösliche Natrium- und/oder Kaliumverbindungen als Hilfsstoff eingesetzt werden.

In einer anderen Ausgestaltungsform des Verfahrens kann als Hilfsstoff Calciumcarbonat, insbesondere Carbokalk, der Fermentationsanlage zugefügt werden.

In einer Ausführungsform des erfindungsgemäßen Verfahrens wird der Fermentationsanlage ein Nährmedium zugegeben, welches ein Substrat und Schlamm umfasst.

In einer weiteren Ausgestaltung wird der Fermentationsanlage 0,1 - 25 Gew.-%, insbesondere 1 - 15 oder 2 - 10 Gew.-% des Hilfsstoffes bezogen auf die eingesetzte Substratmenge zugegeben. Im Unterschied zu bekannten Verfahren wird folglich ein Vielfaches der zum Ausgleich des Kalk-Kohlensäure-Gleichgewichtes benötigten Menge zugesetzt.

Carbokalk wird z. B. bei der Gewinnung von Zucker aus Zuckerrüben gewonnen und stellt folglich ein Nebenprodukt der Zuckerherstellung dar. Dabei wird typischerweise der Zuckerrübenextraktionssaft mit sogenannter Kalkmilch (Suspension aus Calciumhydroxid in Wasser) versetzt. Beim anschließenden Einleiten von Kohlendioxid entsteht Calciumcarbonat, welches ausfällt und dabei Bestandteile des Zuckerrübenextraktionssaftes bindet, wodurch diese entfernbar werden. Bei Verwendung von Filtern, z. B. Vakuumdrehtrommelfiltern, entsteht der sogenannte Scheideschlamm, der jedoch relativ hohe Mengen an Wasser enthält. Bei Verwendung von Kammerfilterpressen zur Abtrennung entsteht ein relativ trockenes Produkt, der Carbokalk. Carbokalk ist somit ein Filterkuchen mit etwa 40 bis 70 Gew.-% Trockensubstanzgehalt, welcher neben Calciumcarbonat bezogen auf die Trockenmasse etwa bis zu 10 Gew.-% organische Bestandteile enthält. Er ist in großer Menge und zu geringem Preis verfügbar.

Carbokalk wird gegenwärtig überwiegend als Kalkdünger verwendet, jedoch sind seine Verwendungsmöglichkeiten auf Gebiete begrenzt, deren Böden von Natur aus keinen ausreichend hohen Calciumgehalt aufweisen.

DE-C-196 14 430 beschreibt ein Verfahren zur Gewinnung von Calciumoxid aus Carbokalk, wobei das hergestellte Calciumoxid zur Reinigung von Zuckerrübenextraktionssaft, zur Rauchgasreinigung oder als Bestandteil von Baukalk eingesetzt werden kann.

In einer Ausführungsform kann das erfindungsgemäße Verfahren zur Herstellung von aus Kohlenstoffverbindungen hergestelltem Gas, insbesondere Biogas, in einer Fermentationsanlage eingesetzt werden, wobei der Hilfsstoff, welcher aus einer Calcium- und/oder Magnesiumverbindung besteht, zur Fermentation zugefügt wird.

In einer weiteren Ausführungsform wird der Fermentationsanlage eine entsprechende Menge an partikulärem, calciumhaltigem Hilfsstoff vor einer ersten Substratzugabe (beim Anfahren des Fermentationsprozesses) zugefügt.

Durch das erfindungsgemäße Verfahren ist eine Erhöhung der Raumzeitausbeute des Fermenters auf mehr als 4 kg/m$^3$*d, insbesondere mehr als 9 kg/m$^3$*d oder mehr als 11 kg/m$^3$*d durch die Beimengung des Hilfsstoffs zur Fütterung möglich. Durch diese überraschende und signifikante Ausbeutesteigerung wird die Rentabilität von Biogasanlagen wesentlich erhöht, wodurch sich die Möglichkeit eröffnet, Biogasanlagen mit kleinerem Reaktorraum zu betreiben. Dies führt auch zu einer höheren Wirtschaftlichkeit.

Vorteilhaft ist ferner eine Erhöhung der Sinkgeschwindigkeit der Partikel und der Mikroorganismen in der Fermentationsanlage durch den im erfindungsgemäßen Verfahren eingesetzten Hilfsstoff, was zu einem geringeren Austrag von Mikroorganismen aus einer Biogasanlage führt.

Dabei kann das Nährmedium Biomasse, wie z. B. Holz, Stroh, Lebensmittelabfällen, Pflanzen oder organischen Reststoffen als Substrat umfassen. Andere kohlenstoffhaltige Quellen sind grundsätzlich auch einsetzbar.

In einer zusätzlichen Ausführungsform des erfindungsgemäßen Verfahrens kann das Nährmedium (oder Impfmedium) weiterhin Schlamm, zum Beispiel Faulschlamm umfassen. Insbesondere können anaerobe Schlämme aus Abwasserreinigungsanlagen von Zuckerfabriken eingesetzt werden.

In noch einer Ausführungsform des erfindungsgemäßen Verfahrens kann die Fermentationsanlage mit diesem Schlamm oder Schlamm aus einem Ablauf einer Fermentationsanlage angeimpft werden. Bei dem anaeroben Schlamm handelt es sich um Biomasse, die bei der anaeroben Aufbereitung von Abwasser entsteht.

Das Hinzufügen von Carbokalk als Hilfsstoff gemäß dem erfindungsgemäßen Verfahren führt vorteilhafterweise zu einer Erhöhung der Sinkgeschwindigkeit der Partikel von Schlamm in der Fermentationsanlage. Dieser Schlamm wird quasi "schwerer" und kann folglich leichter mit einfachen Sedimentationsverfahren abgetrennt, aufkonzentriert und anschließend in den Fermenter oder die Fermentationsanlage zurückgeführt werden. Dadurch lässt sich der Austrag der im Faulschlamm enthaltenen Mikroorganismen verringern.

In einer zusätzlichen Ausgestaltung des erfindungsgemäßen Verfahrens wird ein Teil des Schlamms des Ablaufs in die Fermentationsanlage zurückgeführt, wodurch eine Kreislaufführung des hilfsstoffhaltigen Schlammes ermöglicht wird. Dadurch wird die einzusetzende Menge an Hilfsstoff deutlich verringert.

Der Hilfsstoff bewirkt ferner eine erhöhte Sinkgeschwindigkeit der Partikel im Schlamm und ermöglich dadurch eine vergleichsweise einfache Abtrennung des Schlamms von einer flüssigen Phase zum Beispiel in einem Sedimentationsbecken. Solche Sedimentationsbehälter waren bislang in der Biogaserzeugung nicht einsetzbar, weil sich partikuläre Stoffe im Schlamm nur unvollständig absetzen. Auch andere mechanische Fest-Flüssig-Trennverfahren, wie zum Beispiel eine Siebschneckenpresse oder eine Dekantierzentrifuge, können zu einer verbesserten Sedimentierung führen.
Im Schlamm sind auch die für die Fermentation verantwortlichen Mikroorganismen enthalten. Durch die vereinfachte Abtrennung des Schlamms wird deren Austrag verringert, sie können in die Fermentationsanlage zurückgeführt und somit "wiederverwendet" werden. Dies verringert die einzusetzende Menge an Schlamm bei der Animpfung des Substrates des Nährmediums.

In einer weiteren Ausführungsform wird der Schlamm in einem Sedimentationsverfahren mit oder ohne Flockungshilfsmittel abgetrennt. Unter Flockungshilfsmitteln versteht man dabei Stoffe, die das Zeta-Potential der Teilchen in kolloidalen Suspensionen so beeinflussen, dass sie zu Flocken aggregieren und nach Sedimentation aus dem System entfernt werden können. Die Flockungshilfsmittel müssen dazu die elektrostatische Abstoßung der Partikel überwinden. Neben Naturprodukten wie Stärke haben sich synthetische Polyelektrolyte auf der Basis von Polyacrylamid, Polyacrylat, Polyethylenimin oder Polyethylenoxid durchgesetzt. Durch die Zusammenballung als Flocken können sich die Feststoffe wegen ihrer größeren Masse bedeutend schneller absetzen.

Ein weiterer Vorteil des erfindungsgemäßen Verfahrens ist die gute Lagerung des erfindungsgemäßen Feststoffes oder des nicht mehr biogashaltigen Ablaufs bei sogenannten Stillstandzeiten, d. h. wenn die Fermentationsanlage nicht in Betrieb ist, über Monate oder Jahre hinweg. Dies ist besonders für Anwendungen von Vorteil, bei denen die Biogasproduktion nicht kontinuierlich erfolgt, sondern einem Bedarf angepasst werden muss. Durch diesen Feststoff oder Ablauf ist außerdem ein sehr schnelles Anfahren einer Fermentationsanlage auf Volllastbetrieb innerhalb von beispielsweise ein oder zwei Wochen möglich.

In einer weiteren Ausführungsform wird das erfindungsgemäße Verfahren mesophil (bei einer Temperatur zwischen 20 und 40 °C, insbesondere zwischen 30 und 40 °C oder bei 39 °C) oder thermophil (ab 40 °C, insbesondere zwischen 40 und 85 °C oder 40 und 60° C oder bei 55 °C) durchgeführt.

Das erfindungsgemäße Verfahren kann in der Landwirtschaft, der Lebensmittel- oder Entsorgungsindustrie zur Herstellung von Biogas eingesetzt werden.

In einer Ausführungsform wird das erfindungsgemäße Verfahren zur Fermentation von Zuckerrüben unter Zugabe von Carbokalk als Hilfsstoff durchgeführt.

Da der Hilfsstoff während des Verfahrens nur geringfügig verbraucht wird, ist ein weiterer Gegenstand der Erfindung ein calcium- und/oder magnesiumhaltiger Feststoff, der durch das erfindungsgemäße Verfahren erhältlich ist. Dieser Stoff enthält als sogenannter Gärrest neben den Bestandteilen des Hilfsstoffes (insbesondere Carbokalk) auch anionische Verbindungen (vor allem Stickstoff- und Phosphorverbindungen) und kann somit als Bodenverbesserungsstoff oder als Phosphatdünger in der Landwirtschaft eingesetzt werden.

Als Produkte des erfindungsgemäßen Fermentationsverfahrens entstehen neben Biogas der erfindungsgemäße Feststoff (Gärrest) und eine flüssige Phase. Sowohl der Feststoff, als auch die flüssige Phase enthalten Calcium- und/oder Magnesiumverbindungen bzw. Calcium- und/oder Magnesiumionen, wodurch ein Austrag dieser Stoffe stattfindet. Um den hohen Überschuss an Hilfsstoff in der Fermentationsanlage beizubehalten, ist eine regelmäßige Zugabe erforderlich. Das erfindungsgemäße Verfahren bedingt folglich einen hohen Verbrauch an Hilfsstoff, der den ökonomischen und ökologischen Vorteilen der überraschenden Ausbeutesteigerung an Biogas und der Veredelung des Hilfsstoffes mit den Inhaltsstoffen des Gärrestes gegenübersteht.

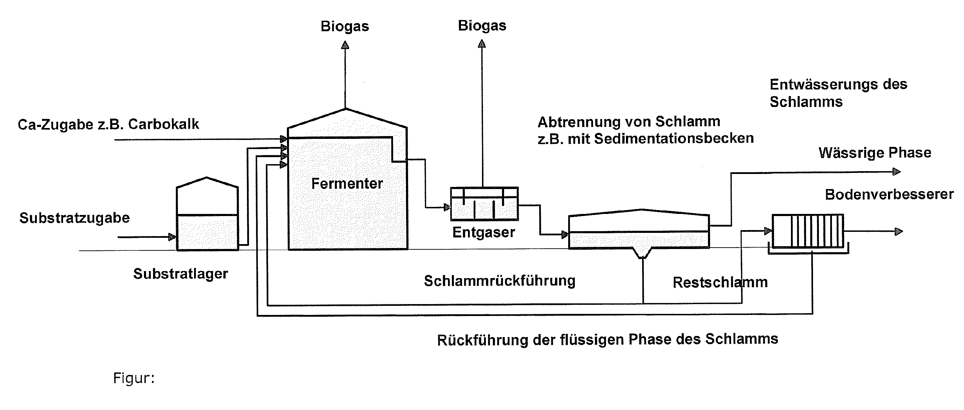

Beschreibung der Figur:

Die Figur zeigt ein Schema des Fermentationsverfahrens. Dabei wird zunächst ein Substrat aus einem Substratlager zusammen mit einem großen Überschuss eines partikulären, calciumhaltigen Hilfsstoffes der gut durchmischten Fermentationsanlage (dem Fermenter) zugegeben, wobei als Substrat auch hydrolysierte, vorversäuerte oder silierte Biomasse denkbar ist. Als Impfmaterial wird ein möglicherweise calciumhaltiger Schlamm zugegeben, wobei es sich um eine flüssige Phase des vorangegangenen Fermentationsprozesses handeln kann. Diese Edukte werden gut durchmischt und zu Biogas und dem sogenannten Ablauf umgesetzt. Aus dem Ablauf wird verbliebenes Biogas in einem Entgaser gewonnen. Hierauf schließt sich eine Aufkonzentrierung des Ablaufs in einem Sedimentationsbecken an, wobei eine erste flüssige Phase ("Wässrige Phase") und ein Schlamm gewonnen wird. Die erste flüssige Phase wird abgetrennt. Ein Teil des Schlammes kann als Impfmaterial in die Fermentationsanlage zurückgeführt werden. Dadurch wird eine Kreislaufführung des calciumhaltigen Schlammes ermöglicht.
Der verbleibende Schlamm (Restschlamm) wird nochmals entwässert. Die entstehende zweite flüssige Phase wird abgetrennt und kann der Fermentationsanlage als Impfmaterial zugegeben werden. Es verbleibt ein nahezu fester Stoff, welcher als Bodenverbesserungsmittel eingesetzt werden kann.

**Beispiel:**

Es werden etwa 1000 kg Zuckerrüben mit Blättern als organisches Substrat und ungefähr 100 kg Carbokalk, insbesondere 80 kg Carbokalk in einen Fermenter gegeben. Als Impfmaterial des Fermentationsprozesses werden etwa 100 kg anaeroben Schlammes einer Abwasserreinigungsanlage hinzugefügt. Im Fermenter erfolgt anschließend die Umsetzung des organischen Substrats zu Biogas (ca. 180 kg) unter anfänglicher Zugabe von Wärme (Aufheizen auf 39 °C bzw. 55 °C). Die Raumzeitausbeute beträgt ungefähr 12 kg/m³*d. Daneben entsteht der Ablauf, welcher ein Gemisch aus calciumhaltigem, anaerobem Faulschlamm (Gärrest) und einer flüssigen Phase, die Calciumionen enthält, umfasst. Der Ablauf wird zunächst entgast, wobei freigesetztes Biogas (ca. 20 kg) gewonnen wird. Hierauf folgt eine Schlammaufkonzentrierung in einem Sedimentationsbecken und dadurch eine Abtrennung einer ersten flüssigen Phase vom verbleibenden Schlamm (ca. 300 kg). Die erste flüssige Phase (etwa 750 kg) wird abgetrennt. Ein Teil des Schlammes (etwa 100 kg) wird als Impfmaterial eines nächsten Fermentationsprozesses in die Fermentationsanlage rückgeführt.

Der Rest des Schlammes (ca. 200 kg; inklusive des Hilfsstoffes) wird z. B. mit Presskammerfiltern mechanisch entwässert, wobei eine zweite flüssige Phase (etwa 50 kg) abgetrennt wird, die ebenfalls in die Fermentationsanlage zurückgeführt wird. Es entsteht ein praktisch fester Stoff (150 kg), der reich an Calcium ist und zum Beispiel als Bodenverbesserungsstoff eingesetzt werden kann.

**Patentansprüche**

1. Verfahren zur Herstellung von Gas aus kohlenstoffhaltigen Materialien in einer Fermentationsanlage, **dadurch gekennzeichnet, dass** ein Hilfsstoff, welcher eine Calcium- und/oder Magnesiumverbindung umfasst, zugegeben wird.

2. Verfahren gemäß Anspruch 1, **dadurch gekennzeichnet, dass** die Calcium- und/oder Magnesiumverbindung nicht leichtlöslich ist.

3. Verfahren gemäß Anspruch 1 und/oder 2, **dadurch gekennzeichnet, dass** die Calciumverbindung Calciumcarbonat, insbesondere Carbokalk ist.

4. Verfahren gemäß mindestens einem der Ansprüche 1-3, **dadurch gekennzeichnet, dass** der Hilfsstoff im Überschuss zugegeben wird.

5. Verfahren gemäß mindestens einem der Ansprüche 1 - 4, **dadurch gekennzeichnet, dass** es sich bei dem Gas um Biogas handelt.

6. Verfahren gemäß mindestens einem der Ansprüche 1 - 5, **dadurch gekennzeichnet, dass** die Raumzeitausbeute mehr als 4 kg/m³*d, insbesondere mehr als 9 kg/m³*d oder mehr als 11 kg/m³*d beträgt.

7. Verfahren gemäß mindestens einem der Ansprüche 1 - 6, **dadurch gekennzeichnet, dass** der Fermentationsanlage ein Nährmedium zugegeben wird, welches ein Substrat und Schlamm umfasst.

8. Verfahren gemäß Anspruch 7, **dadurch gekennzeichnet, dass** es sich bei dem Schlamm um Faulschlamm handelt, welcher als Impfmaterial dient.

9. Verfahren gemäß mindestens einem der Ansprüche 1 - 8, **dadurch gekennzeichnet, dass** der Hilfsstoff die Sinkgeschwindigkeit von Partikeln im Schlamm erhöht und den Austrag von Mikroorganismen aus der Fermentationsanlage verringert.

10. Verfahren gemäß Anspruch 9, **dadurch gekennzeichnet, dass** der Schlamm in einem Sedimentationsverfahren mit oder ohne Flockungshilfsmittel abgetrennt werden kann.

11. Verfahren gemäß mindestens einem der Ansprüche 1-10, **dadurch gekennzeichnet, dass** die Fermentationsanlage eine Biogasanlage ist.

12. Verfahren gemäß mindestens einem der Ansprüche 1-11, **dadurch gekennzeichnet, dass** das Verfahren thermophil oder mesophil durchgeführt wird.

13. Verwendung des Verfahrens gemäß Anspruch 1 zur Herstellung von Gas aus kohlenstoffhaltigen Materialien, insbesondere Biogas, in der Landwirtschaft, Lebensmittel- oder Entsorgungsindustrie.

14. Calcium- und/oder magnesiumhaltiger Feststoff, **dadurch gekennzeichnet, dass** er durch das Verfahren gemäß Anspruch 1 erhältlich ist und als Gärrest anionische Verbindungen (vor allem Stickstoff- und Phosphorverbindungen) umfasst.

15. Verwendung des Feststoffes gemäß Anspruch 13 als Bodenverbesserungsstoff oder als Phosphatdünger in der Landwirtschaft.

Biogas

Biogas

Entwässerungs des Schlamms

Ca-Zugabe z.B. Carbokalk

Abtrennung von Schlamm z.B. mit Sedimentationsbecken

Wässrige Phase

Fermenter

Bodenverbesserer

Substratzugabe

Entgaser

Substratlager

Schlammrückführung

Restschlamm

Rückführung der flüssigen Phase des Schlamms

Figur:

Europäisches Patentamt
European Patent Office
Office européen des brevets

**EUROPÄISCHER RECHERCHENBERICHT**

Nummer der Anmeldung

EP 08 10 1684

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DATABASE WPI Week 198132 Thomson Scientific, London, GB; AN 1981-57533D XP002488364 & JP 56 073599 A (MORINAGA ENGINEERING KK) 18. Juni 1981 (1981-06-18) * Zusammenfassung * ----- | 1-13 | INV. C12P5/02 |
| X | DATABASE WPI Week 198842 Thomson Scientific, London, GB; AN 1988-295343 XP002488365 & JP 63 214399 A (MATSUSHITA ELECTRIC IND CO LTD) 7. September 1988 (1988-09-07) * Zusammenfassung * ----- | 1-13 | |
| X | DATABASE WPI Week 198736 Thomson Scientific, London, GB; AN 1987-254451 XP002488366 & JP 62 176596 A (EBARA INFILCO KK) 3. August 1987 (1987-08-03) * Zusammenfassung * ----- | 1-13 | |
| X | WANG, X. ET AL.: "Optimization of methane fermentation from effluent of bio-hydrogen fermentation process using response surface methodology" BIORESOURCE TECHNOLOGY, Bd. 99, Nr. 10, 24. Oktober 2007 (2007-10-24), Seiten 4292-4299, XP022526240 * das ganze Dokument * siehe insbesondere: * Zusammenfassung * * Seite 4293, Spalte 1, Zeilen 30-36 * * Seite 4298, Spalte 2, Zeilen 22-24 * ----- -/-- | 1-13 | RECHERCHIERTE SACHGEBIETE (IPC) C12P C02F C05F |

~~Der vorliegende Recherchenbericht wurde für alle Patentans~~prüche erstellt

| Recherchenort | Abschlußdatum der Recherche | Prüfer |
|---|---|---|
| Berlin | 16. Juli 2008 | Fuchs, Ulrike |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

EP 2 090 660 A1

**Europäisches Patentamt**
**European Patent Office**
**Office européen des brevets**

# EUROPÄISCHER RECHERCHENBERICHT

**Nummer der Anmeldung**

EP 08 10 1684

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 496 019 A (EBARA CORPORATION) 12. Januar 2005 (2005-01-12) * Seite 2, Absatz 1-3 * * Seite 4, Absatz 16 * * Seite 6, Absatz 32 * * Seite 13; Abbildung 1 * ----- | 1-13 | |
| D,A | EP 0 934 998 A (HOFFMANN, M.; INSTITUT F. AGRARTECHNIK BORNIM E.V. ATB) 11. August 1999 (1999-08-11) * das ganze Dokument * siehe insbesondere: * Spalten 12-13; Ansprüche 1,4,7,10 * ----- | 1-13 | |

**RECHERCHIERTE SACHGEBIETE (IPC)**

~~Der vorliegende Recherchenbericht wurde für alle Patentans~~prüche erstellt

| Recherchenort | Abschlußdatum der Recherche | Prüfer |
|---|---|---|
| Berlin | 16. Juli 2008 | Fuchs, Ulrike |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
................................................................................................
& : Mitglied der gleichen Patentfamilie, übereinstimmendes
    Dokument

EPO FORM 1503 03.82 (P04C03)

**Europäisches Patentamt**
**European Patent Office**
**Office européen des brevets**

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

1-13 (vollständig)

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches
Patentamt

European
Patent Office

Office européen
des brevets

**MANGELNDE EINHEITLICHKEIT
DER ERFINDUNG
ERGÄNZUNGSBLATT B**

**Nummer der Anmeldung**

EP 08 10 1684

Nach Auffassung der Recherchenabteilung entspricht die vorliegendeeuropäische Patentanmeldung nicht den Anforderungen an die Einheitlichkeit der Erfindungund enthält mehrere Erfindungen oder Gruppen von Erfindungen, nämlich:

1. Ansprüche: 1-13 (vollständig)

   Verfahren zur Herstellung von Gas aus kohlenstoffhaltigen Materialien in einer Fermentationsanlage, dadurch gekennzeichnet, dass ein Hilfsstoff, welcher eine Calcium- und/oder Magnesiumverbindung umfasst, zugegeben wird
   ---

2. Ansprüche: 14, 15 (vollständig)

   Calcium- und/oder magnesiumhaltiger Feststoff erhältlich durch besagtes Verfahren, dadurch gekennzeichnet, dass er als Gärrest anionische Verbindungen (vor allem Stickstoff- und Phosphorverbindungen) umfasst
   ---

**ANHANG ZUM EUROPÄISCHEN RECHERCHENBERICHT**
**ÜBER DIE EUROPÄISCHE PATENTANMELDUNG NR.**

EP 08 10 1684

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-07-2008

| Im Recherchenbericht angeführtes Patentdokument | | Datum der Veröffentlichung | Mitglied(er) der Patentfamilie | | Datum der Veröffentlichung |
|---|---|---|---|---|---|
| JP 56073599 | A | 18-06-1981 | KEINE | | |
| JP 63214399 | A | 07-09-1988 | KEINE | | |
| JP 62176596 | A | 03-08-1987 | JP | 1916360 C | 23-03-1995 |
| | | | JP | 6030773 B | 27-04-1994 |
| EP 1496019 | A | 12-01-2005 | CN | 1606533 A | 13-04-2005 |
| | | | WO | 03086990 A1 | 23-10-2003 |
| | | | US | 2005016919 A1 | 27-01-2005 |
| EP 0934998 | A | 11-08-1999 | AT | 308608 T | 15-11-2005 |
| | | | DE | 19805045 A1 | 12-08-1999 |
| | | | DE | 59912725 D1 | 08-12-2005 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0934998 B

- DE 19614430 C

**In der Beschreibung aufgeführte Nicht-Patentliteratur**

- **Mudrack K. ; Kunst, S.** Biologie der Abwasserreinigung. Spektrum-Akademischer Verlag, 2003